(19) Europäisches Patentamt
European Patent Office
Office européen des brevets

(11) **EP 2 364 640 A1**

(12) **EUROPÄISCHE PATENTANMELDUNG**

(43) Veröffentlichungstag:
**14.09.2011 Patentblatt 2011/37**

(51) Int Cl.:
***A61B 5/0205*** *(2006.01)*    ***A61N 1/36*** *(2006.01)*
***A61N 1/38*** *(2006.01)*

(21) Anmeldenummer: **11155185.9**

(22) Anmeldetag: **21.02.2011**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **11.03.2010   US 312685 P**

(71) Anmelder: **BIOTRONIK SE & Co. KG**
**12359 Berlin (DE)**

(72) Erfinder:
• **Lippert, Michael**
**91522, Ansbach (DE)**

• **Vollkron, Michael**
**3021, Pressbaum (AT)**
• **Czygan, Gerald**
**91054, Buckenhof (DE)**
• **Paule, Stefan**
**96117, Drosendorf (DE)**

(74) Vertreter: **Lindner-Vogt, Karin L.**
**Biotronik SE & Co. KG**
**Corporate Intellectual Properties**
**Woermannkehre 1**
**12359 Berlin (DE)**

(54) **Überwachungsvorrichtung und Verfahren zum Betreiben einer Überwachungsvorrichtung**

(57)    Die vorliegende Erfindung betrifft eine Überwachungsvorrichtung (100) für einen Patienten zum Vorhersagen einer kardiovaskulären Unregelmäßigkeit sowie ein Verfahren (300) zum Betreiben einer Überwachungsvorrichtung (100). Ferner betrifft die vorliegende Erfindung eine implantierbare Elektrotherapievorrichtung, wie beispielsweise ein implantierbarer Herzschrittmacher (200), ein implantierbarer Kardioverter (200) oder ein implantierbarer Defibrillator (200), mit einer erfindungsgemäßen Überwachungsvorrichtung (100). Die erfindungsgemäße Überwachungsvorrichtung (100) erfasst in einer Ausführungsform eine Wertänderung eines hämodynamischer Parameters, die in Folge einer detektierten Wertänderung eines Zustandsparameters, wie beispielsweise in Folge eines Aktivierens oder Deaktivierens einer kardialen Resynchronisationstherapie, stattfindet. Durch geeignete Bewertung der Wertänderung des hämodynamischen Parameters kann die Überwachungsvorrichtung (100) ein für ein Anbahnen einer kardiovaskulären Unregelmäßigkeit, wie beispielsweise eine kardiale Dekompensation, indikatives Bewertungsergebnissignal lange im Voraus und mit hoher Spezifität ausgeben.

**FIG. 1**

**Beschreibung**

Die vorliegende Erfindung betrifft eine Überwachungsvorrichtung für einen Patienten zum Vorhersagen einer kardiovaskulären Unregelmäßigkeit sowie ein Verfahren zum Betreiben einer Überwachungsvorrichtung. Ferner betrifft die vorliegende Erfindung eine implantierbare Elektrotherapievorrichtung, wie beispielsweise ein implantierbarer Herzschrittmacher, ein implantierbarer Kardioverter oder ein implantierbarer Defibrillator, die mit einer erfindungsgemäßen Überwachungsvorrichtung wirkverbunden ist.

Insbesondere betrifft die vorliegende Erfindung einen Kardioverter/Defibrillator, die mit einer erfindungsgemäßen Überwachungsvorrichtung zur Vorhersage einer kardialen Dekompensation wirkverbunden ist.

Überwachungsvorrichtungen sowie Verfahren zum Vorhersagen einer kardiovaskulären Unregelmäßigkeit sind grundsätzlich bekannt.

Beispielsweise ist in der Veröffentlichung der amerikanischen Patentanmeldung US 2005/0124900 A1 ein Verfahren und eine Vorrichtung zum Detektieren von Wertänderungen physiologischer Parameter und darauf basierenden Vorhersagen einer kardiovaskulären Unregelmäßigkeit beschrieben. Bei diesem Verfahren wird ein Wert eines physiologischen Parameters, wie beispielsweise ein Druck oder eine Herzratenvariabilität, gemessen. Der gemessene Wert wird dann zum einen über eine vergleichsweise lange Zeitperiode gemittelt und zum anderen über eine vergleichsweise kurze Zeitperiode gemittelt. Der über die vergleichsweise lange Zeitperiode gemittelte Wert des physiologischen Parameters repräsentiert einen gesunden Zustand des Patienten. Ferner wird die Abweichung des kurzzeitgemittelten Werts vom langzeitgemittelten Wert aufgezeichnet und die Werte der relativen Abweichung kontinuierlich integriert. Ein für eine kardiovaskuläre Unregelmäßigkeit indikatives Alarmsignal wird entweder dann ausgegeben, wenn die relative Abweichung einen ersten vorgegebenen Wert überschreitet oder wenn die integrierte relative Abweichung einen zweiten vorgegebenen Wert überschreitet.

Es ist ein der vorliegenden Erfindung zugrundeliegendes technisches Problem, eine Überwachungsvorrichtung zum Vorhersagen einer kardiovaskulären Unregelmäßigkeit vorzuschlagen, die sich insbesondere durch die Fähigkeit auszeichnet, eine kardiovaskuläre Unregelmäßigkeit möglichst lange im Voraus und mit hoher Spezifität ankündigen zu können.

Gemäß einem ersten Aspekt der vorliegenden Erfindung wird das oben genannte technische Problem gelöst für eine Überwachungsvorrichtung für einen Patienten zum Vorhersagen einer kardiovaskulären Unregelmäßigkeit, die folgende Komponenten umfasst:

- einen ersten Eingang und einen mit dem ersten Eingang verbundenen Sensor zum Erfassen eines Wertes eines von einer kardiovaskulären Unregelmäßigkeit abhängigen physiologischen Parameters, wobei der erste Eingang und der Sensor implantierbar sind,

- einen zweiten Eingang und eine mit dem zweiten Eingang verbundene Detektionseinheit zum Detektieren einer Wertänderung eines vom physiologischen Parameter verschiedenen Zustandsparameters, wobei der Zustandsparameter ein anderer physiologischer Parameter oder ein einen Zustand einer mit dem Patienten bereits verbundenen medizinischen Vorrichtung charakterisierender Parameter oder ein einen sonstigen physischen oder pharmakotherapeutischen Zustand des Patienten charakterisierender Parameter ist,

- eine mit dem implantierbaren Sensor und der Detektionseinheit wirkverbundene Auswerteeinheit, die ausgebildet ist, auf die Detektion der Wertänderung des Zustandsparameters hin eine auf die Wertänderung des Zustandsparameters hin erfolgende Wertänderung des physiologischen Parameters zu bestimmen und in Abhängigkeit der bestimmten Wertänderung des physiologischen Parameters ein erstes Differenzsignal bereitzustellen,

- eine Transformationseinheit, die ausgebildet ist, das erste Differenzsignal zu empfangen und in Abhängigkeit einer zeitlichen Korrelation der Wertänderungen des Zustandsparameters und des physiologischen Parameters und/oder in Abhängigkeit eines Betrages und/oder einer Richtung der detektierten Wertänderung des Zustandsparameters in eine Kenngröße zu transformieren und bereitzustellen,

- eine Vorhersageeinheit, die ausgebildet ist, die Kenngröße zu empfangen und zu bewerten sowie in Abhängigkeit der Bewertung ein für ein Anbahnen einer kardiovaskulären Unregelmäßigkeit indikatives Bewertungsergebnissignal bereitzustellen.

Die vorliegende Erfindung beruht auf der Erkenntnis, dass bekannte Vorrichtungen zum Vorhersagen einer kardiovaskulären Unregelmäßigkeit, wie beispielsweise zum Vorhersagen einer kardialen Dekompensation, eines Herzinfarkts, eines Schlaganfalls, einer Verschlechterung einer Herzinsuffizienz, einer sonstigen Fehlfunktion des Herz-Kreislauf-Systems, lediglich den Verlauf eines einzigen physiologischen Parameters betrachten. Derartige Vorrichtungen neigen dazu, Fehlalarme auszugeben, da fast alle physiologischen Parameter auch Wertänderungen unterliegen, die nicht direkt auf eine Verschlechterung des Herz-Kreislauf-Systems zurückzuführen sind, sondern andere Ursachen haben. Daher weisen derartige Vorrichtungen eine vergleichsweise niedrige Spezifität auf, was generell nachteilig ist.

Beispielsweise kann zum Vorhersagen einer kardiovaskulären Unregelmäßigkeit die Pumpleistung des Herzens anhand einer Werterfassung und -auswertung eines hämodynamischen Parameters erfolgen. Das Auswerten des Verlaufs eines Wertes eines solchen hämodynamischen Parameters wird jedoch durch folgende von den Erfindern erkannte Problematik erschwert: Einem Verschlechtern der Pumpfunktion des Herzens begegnet der Körper mit einer Vielzahl verschiedener Kompensationsmechanismen, wie beispielsweise einem Variieren des Füllungsdrucks, des peripheren Widerstandes, der Gesamtflüssigkeitsmenge, der Herzrate, der Kontraktilität. Aufgrund eines solchen Variierens wird die Pumpleistung des Herzens zunächst aufrechterhalten. Beispielsweise wird durch ein Erhöhen des Füllungsdruckes des Ventrikels das Schlagvolumen des Herzens zunächst konstant gehalten. Erst in einer späten Phase, wenn körpereigene Kompensationsmechanismen gänzlich ausgereizt sind, sinkt die Pumpleistung selbst. Daher lässt sich beispielsweise durch alleiniges Überwachen des Schlagvolumens des Herzens eine kardiovaskuläre Unregelmäßigkeit anhand einer ermittelten Änderung des Schlagvolumens des Herzens erst relativ spät ankündigen. Somit lässt sich eine akzeptable Spezifität bei bekannten Vorrichtungen nur durch eine Einbuße in der Vorwarnzeit erzielen.

Weiterhin haben die Erfinder erkannt, dass andere bekannte Vorrichtungen zur Vorhersage einer kardiovaskulären Unregelmäßigkeit, die auf einem Detektieren von Lungenwasserbildung beruhen, einen Alarm grundsätzlich erst kurz vor einem Ausbilden klinischer Symptome ausgeben, womit auch die Vorwarnzeit dieser Vorrichtungen gering ist. Außerdem bedingt nicht jede Verschlechterung des Herz-Kreislauf-Systems ein Bilden von Lungenwasser, was bei Überwachungsvorrichtungen, die ausschließlich auf der Detektion von Lungenwasser beruhen, folglich zu einem Ausbleiben von Alarmen führen kann.

Die vorliegende Erfindung beruht auf der weiteren Erkenntnis, dass es zum Erzielen einer längeren Vorwarnzeit zweckmäßig ist, Werte eines oder mehrerer physiologischer Parameter zu erfassen, die sich vergleichsweise früh in der Wirkungskette einer kardiovaskulären Unregelmäßigkeit ändern.

Ferner und insbesondere haben die Erfinder beobachtet, dass das Herz-Kreislauf-System auf eine Wertänderung eines bestimmten Zustandsparameters, die ein Stören des Herz-Kreislauf-Systems bewirkt, anders reagiert, wenn sich eine kardiovaskuläre Unregelmäßigkeit anbahnt. Dieses abweichende Reagieren des Herz-Kreislauf-Systems, welches sich beispielsweise in einer Änderung der Stärke oder der Geschwindigkeit der Reaktion äußert, ist darauf zurückzuführen, dass die Möglichkeiten zur Kompensation der Störung aufgrund des Anbahnens der kardiovaskulären Unregelmäßigkeit bereits verringert sind. Das abweichende Reagieren lässt sich beispielsweise bei der Herzratenvariabilität (HRV) beobachten. Regelungstechnisch ausgedrückt lässt sich das Herz-Kreislauf-System demnach als ein Regelkreis beschreiben, dessen Regelgröße in Abhängigkeit eines Anbahnens einer kardiovaskulären Unregelmäßigkeit auf eine Wertänderung einer Störgröße verschiedenartig reagiert.

Es ist demnach ein Grundgedanke der vorliegenden Erfindung, nicht lediglich einen Wert eines physiologischen Parameters kontinuierlich aufzuzeichnen und zu bewerten, sondern eine Wertänderung des physiologischen Parameters zu bestimmen, die sich infolge einer Wertänderung eines Zustandsparameters ergibt sowie die Wertänderung des physiologischen Parameters in Relation zu setzen mit der Wertänderung des Zustandsparameters und so eine Kenngröße zu gewinnen, die als Basis für eine langfristige und spezifische Vorhersage einer kardiovaskulären Unregelmäßigkeit dient.

Aufgrund der Bewertung der Kenngröße, die sich aus der Wertänderung des physiologischen Parameters und wahlweise der zeitlichen Korrelation der Wertänderung des Zustandsparameters und des physiologischen Parameters und/oder des Betrages und/oder der Richtung der detektierten Wertänderung des Zustandsparameters zusammensetzt, ist die Überwachungsvorrichtung fähig, eine kardiovaskuläre Unregelmäßigkeit zum einen lange im Voraus und zum anderen mit einer hohen Spezifität vorherzusagen. Selbstredend profitieren von der langen Vorwarnzeit sowohl der Patient als auch ein behandelnder Arzt unmittelbar.

Ein weiterer Vorteil der erfindungsgemäßen Überwachungsvorrichtung ist es, dass langfristige Änderungen des Wertes des physiologischen Parameters, die nicht unmittelbar auf eine Verschlechterung des Herz-Kreislauf-Systems des Patienten zurückzuführen sind, nicht zum Ausgeben eines Alarms im Sinne eines eine kardiovaskuläre Unregelmäßigkeit ankündigenden Bewertungsergebnissignals führen, was die Spezifität der erfindungsgemäßen Überwachungsvorrichtung weiter erhöht.

Ein anderer Vorteil der erfindungsgemäßen Überwachungsvorrichtung ist es, dass die Kenngröße nicht nur dafür verwendet werden kann, eine kardiovaskuläre Unregelmäßigkeit vorherzusagen, sondern auch als Basis für eine zweite Auswertung dienen kann, mit Hilfe der sich Soll-Wertänderungen des physiologischen und/oder des Zustandsparameters berechnen lassen, die einer sich anbahnenden kardiovaskulären Unregelmäßigkeit entgegenwirken. Derartige Soll-Wertänderungen könnten beispielsweise durch die Überwachungsvorrichtung selbst verursacht werden oder einem behandelnden Arzt beispielsweise via Home-Monitoring (HM, Home-Monitoring bezeichnet eine Patientenferndiagnose) vorgeschlagen werden.

Die Komponenten der Überwachungsvorrichtung sind nicht unbedingt allesamt in einem implantierten Gehäuse integriert, sondern können auch räumlich verteilt angeordnet sein. Bevorzugt sind beispielsweise der erste Eingang und der Sensor der Überwachungsvorrichtung implantiert, die Detektionseinheit mit dem zweiten Eingang in Abhängigkeit des Typs des Zustandsparameters wahlweise ebenfalls implantiert oder außerhalb des Körpers des Patienten angeordnet und die übrigen Komponenten, nämlich die Auswerteeinheit, die Transformationseinheit und die Vorhersageeinheit außerhalb des Körpers des Patienten, beispielsweise in einem Patientengerät oder in einem Home-Monitoring-Center, befindlich. Demnach sind jeweilige Komponenten der Überwachungsvorrichtung wahlweise über Leitungen und/oder kabellos miteinander wirkverbunden. Der implantierte Sensor und die Detektionseinheit senden beispielsweise ihre jeweiligen Signale über geeignete Schnittstellen zur Auswerteeinheit.

Wie bereits oben erläutert, steht der Begriff "kardiovaskuläre Unregelmäßigkeit" im Rahmen der Beschreibung der vorliegenden Erfindung stellvertretend für eine Reihe von Herz-Kreislauf-Fehlfunktionen. Darunter fallen beispielsweise eine kardiale Dekompensation, ein Herzinfarkt, ein Schlaganfall oder eine akute Verschlechterung des Herzzustandes, beispielsweise im Rahmen einer Herzinsuffizienz, durch Ausbilden einer Herzklappeninsuffizienz, einer Perikardtamponade, einer Myokarditis oder durch deutliche Herzrhythmusstörungen.

Der von einer solchen kardiovaskulären Unregelmäßigkeit abhängige physiologische Parameter ist beispielsweise ein hämodynamischer Parameter, wie ein Druck, beispielsweise ein pulmonal-arterieller Druck (PAP), ein rechtsventrikulärer Druck (RVP), ein linksventrikulärer Druck (LVP), ein Aortendruck (AoP), ein Druck im linken Vorhof (LAP), ein Druck im rechten Vorhof (RAP), ein Blutdruck, oder ein Blutfluss, ein Volumen, oder eine Beschleunigung, ein biochemischer Parameter, eine Kontraktilität, eine Impedanz oder ein Elektrokardiogramm. Wahlweise können anstelle dieser physiologischen Parameter auch physiologische Surrogatparameter verwendet werden, die z.B. aus einer geeigneten Impedanzmessung gewonnen werden. Im Rahmen der Beschreibung der vorliegenden Erfindung umfasst der Begriff "physiologischer Parameter" ebenfalls physiologische Surrogatparameter.

Das von der Auswerteeinheit in Abhängigkeit der bestimmten Wertänderung des physiologischen Parameters bereitgestellte Differenzsignal gibt wahlweise die bestimmte Wertänderung des physiologischen Parameters selbst an oder eine daraus abgeleitete Wertänderung eines weiteren physiologischen Parameters, wie beispielsweise das Schlagvolumen (SV), das links- oder rechtsventrikuläre enddiastolische Volumen (LVEDV oder RVEDV), das links- oder rechtsventrikuläre endsystolische Volumen (LVESV oder RVESV), die linksventrikuläre Ejektionsfraktion (LVEF), die auf die zeitbezogene links- oder rechtsventrikuläre Druckänderung (RV $dP/dt_{max}$ oder LV $dP/dt_{max}$), der linksventrikuläre enddiastolische Druck (LVEDP), der mittlere oder enddiastolische Druck im linken Vorhof (LAP), der mittlere oder systolische oder diastolische Aortendruck (mean/syst/diast AoP), die linksventrikuläre Kontraktilität, der Lungenwiderstand, ein peripherer Widerstand, eine Gefäß-Compliance oder entsprechende Surrogat-Parameter oder ein T-Wellen-Alternans-Parameter, wie ein ABAB-Muster (ein alternierend wiederkehrendes Muster), die ST-Streckenanhebung, ein anderer morphologischer Elektrokardiogramm-Parameter, ein aus einem Druckvolumendiagramm abgeleiteter Parameter, wie die potenzielle Energie (PE), die externe Arbeit (EW), die Druckvolumenfläche (PVA), die effektive arterielle Elastizität (EA), die Kontraktilität ($E_{max}$), die Effizienz der linken oder rechten Herzhälfte (EW/PE), die sogenannte Pre-Load Recruitable Stroke Work (PRSW) oder ein entsprechender Surrogat-Parameter. Die bestimmte Wertänderung oder die abgeleitete Wertänderungen sind bevorzugt zeitlich gemittelte Werte.

Der Zustandsparameter ist beispielsweise ein einen Zustand einer mit dem Patienten bereits verbundenen medizinischen Vorrichtung charakterisierender Parameter, wie beispielsweise ein Stimulationsparameter, wie eine Stimulationsfrequenz, eine Aktivität einer kardialen Resynchronisationstherapie (CRT on/off), eine Aktivität einer antitachykarden Stimulation (ATP), eine Elektrodenkonfiguration, ein Pacing-Modus oder eine Pacing-Amplitude, eine programmierte atrio- oder interventrikuläre Verzögerungszeit, ein Stimulationsmodus.

Der Zustandsparameter ist in anderen Ausführungsformen ein anderer physiologischer Parameter, wie eine atrioventrikuläre Verzögerungszeit (AVD), eine interventrikuläre Verzögerungszeit (VVD), eine intrinsische Frequenz des Herzens, ein atrialer oder ventrikulärer Ereignistyp, welcher stimuliert oder intrinsisch (also natürlichen Ursprungs) sein kann, oder ein für ein Vorhandensein einer Arrhythmie, eines Vorhofflimmerns (AF), einer supraventrikulären Tachykardie (SVT), einer ventrikulären oder atrialen Extrasystole oder eines sonstigen abweichenden einzelnen Herzzykluses kennzeichnender Parameter, wie beispielsweise die ersten Zyklen nach einem Defibrillationsschock.

Der Zustandsparameter kann auch ein für einen physischen oder pharmakotherapeutischen Zustand des Patienten charakterisierender Parameter sein, wie beispielsweise eine Belastung, die beispielsweise über einen sogenannten Motion-Sensor, über ein Minutenvolu-men-(MV-)Sensor oder über eine Aktivität einer Closed-Loop-Stimulation (CLS) erfasst wird, eine Atemfrequenz oder ein Parameter, der kennzeichnend dafür ist, ob sich der Patient in einem Schlaf- oder Wachzustand befindet, ob Arrhythmien bestehen oder nicht, ob der Patient liegt oder steht, ob der Patient Medikamente, wie beispielsweise Diuretika, einnimmt oder nicht, ob ein Atmungsstillstand (Apnoe) besteht oder nicht.

Beispielsweise stellt also ein Aktivieren oder Deaktivieren einer CRT, das Ansteigen oder Abfallen der Herzfrequenz, ein Ändern der Körperlage des Patienten, die Einnahme von Medikamenten oder das Absetzen von Medikamenten, eine nervale Stimulation, wie beispielsweise eine vagale Stimulation, eine Wertänderung des Zustandsparameters dar. Mit der Wertänderung des Zustandsparameters geht demnach in der Regel eine Störung des Herz-Kreislauf-Systems einher oder die Wertänderung des Zustandsparameters quantifiziert bereits eine Störung des Herz-Kreislauf-Systems.

Die Wertänderung des Zustandsparameters kann selbständig erfolgen oder zum Zwecke der Überwachung provoziert werden, beispielsweise durch die Überwachungsvorrichtung selbst, wozu später noch detaillierter ausgeführt wird. Im letzteren Fall ist die Überwachungsvorrichtung nicht nur ausgebildet, die Wertänderung des Zustandsparameters zu detektieren, sondern diese vorerst zu verursachen.

Die Transformationseinheit ist ausgebildet, das erste Differenzsignal in Abhängigkeit der zeitlichen Korrelation der Wertänderungen des Zustandsparameters und des physiologischen Parameters und/oder in Abhängigkeit eines Betrages und/oder einer Richtung der detektierten Wertänderung des Zustandsparameters in eine Kenngröße zu transformieren. Beispielsweise bezieht die Transformationseinheit das erste Differenzsignal auf die Dauer der Wertänderung des physiologischen Parameters oder auf die Dauer der Wertänderung des Zustandsparameters oder auf eine Zeitdauer zwischen der Wertänderung des Zustandsparameters und der Wertänderung des physiologischen Parameters. In dieser Ausführungsform ist die Kenngröße demnach bezeichnend für eine Dynamik der Wertänderung des physiologischen Parameters infolge der Wertänderung des Zustandsparameters. In einer anderen Ausführungsform bezieht die Transformationseinheit das erste Differenzsignal auf einen Betrag der Wertänderung des Zustandsparameters und/oder auf eine Richtung der Wertänderung des Zustandsparameters, womit die Kenngröße eine gewichtete Stärke oder eine Richtung der Wertänderung des physiologischen Parameters in Reaktion auf die Wertänderung des Zustandsparameters oder eine Empfindlichkeit des physiologischen Parameters in Bezug auf die Wertänderung des Zustandsparameters darstellt.

Die Vorhersageeinheit ist ausgebildet, die Kenngröße zu empfangen und zu bewerten. Das Bewerten der Kenngröße erfolgt beispielsweise durch ein Vergleichen der Kenngröße mit bereits gespeicherten Vergleichswerten. Die gespeicherten Vergleichswerte können dabei vordefiniert sein oder aufgrund der langfristig beobachteten statistischen Schwankungen der Kenngröße adaptiv bestimmt werden. Es kann auch mithilfe statistischer Methoden die Wahrscheinlichkeit für einen Trend in den aufeinander folgenden Werten der Kenngröße bestimmt, und diese Trend-Wahrscheinlichkeit mit einem Grenzwert verglichen werden. Zur weiteren Verminderung von Fehlalarmen kann diese Bewertung über ein Zeitintervall von mehreren Tagen bis Wochen zusammenfassend betrachtet werden.

Nachfolgend werden weitere Ausführungsbeispiele der erfindungsgemäßen Überwachungsvorrichtung beschrieben. Zusätzliche Merkmale der weiteren Ausführungsbeispiele können miteinander sowie mit oben bereits beschriebenen optionalen Merkmalen zur Bildung anderer Ausführungsformen miteinander kombiniert werden, sofern diese nicht explizit als alternativ zueinander beschrieben sind.

In einer bevorzugten Ausführungsform ist die Auswerteeinheit der Überwachungsvorrichtung ausgebildet, die Wertänderung des Zustandsparameters zu bestimmen und in Abhängigkeit der bestimmten Wertänderung des Zustandsparameters ein zweites Differenzsignal bereitzustellen. In dieser Ausführungsform ist die Transformationseinheit ausgebildet, einen Quotienten aus dem ersten Differenzsignal und dem zweiten Differenzsignal als die Kenngröße bereitzustellen.

Das zweite Differenzsignal gibt entweder die bestimmte Wertänderung des Zustandsparameters selbst an oder eine daraus abgeleitete Wertänderung. Beispielsweise transformiert die Transformationseinheit das erste Differenzsignal gemäß Gleichung 1 in die Kenngröße:

$$\text{Kenngröße} = \frac{\Delta(\text{physiologischer Parameter})}{\Delta(\text{Zustandsparameter})} \qquad , \qquad\qquad (1)$$

wobei mit $\Delta$(physiologischer Parameter) die Wertänderung des physiologischen Parameters und mit $\Delta$(Zustandsparameter) die Wertänderung des Zustandsparameter bezeichnet ist.

In dieser Ausführungsform stellt Kenngröße demnach eine Empfindlichkeit des physiologischen Parameters in Bezug auf eine Wertänderung des Zustandsparameters dar. Diese Ausführungsform bietet sich insbesondere dann an, wenn der Zustandsparameter ein anderer physiologischer Parameter, wie beispielsweise die Herzfrequenz, die Belastung, die Atemfrequenz, die atrioventrikuläre Verzögerungszeit (AVD) ist.

Insbesondere in der oben genannten Ausführungsform ist die Auswerteeinheit bevorzugt dazu ausgebildet, kontinuierlich den Wert des physiologischen Parameters aufzuzeichnen und für kurze Zeit zeitlich zu mitteln sowie den kurzzeitgemittelten Wert des physiologischen Parameters zu speichern und kontinuierlich zu aktualisieren und die Wertänderung des physiologischen Parameters als Differenz zwischen einem aktuell erfassten Wert des physiologischen Parameters und dem zuvor gespeicherten Wert des physiologischen Parameters, also dem zeitlich vor der Wertänderung des Zustandsparameters gespeicherten Wert des physiologischen Parameters, zu bestimmen.

Bevorzugt ist die Auswerteeinheit dazu ausgebildet, eine Mittelung des Wertes des physiologischen Parameters in bestimmten vordefinierten Wertbereichen, die im Folgenden auch als Bins bezeichnet werden, des Zustandsparameters, wie beispielsweise im Falle einer Herzfrequenz als Zustandsparameters in den Wertbereichen:

40-60 bpm (beats per minute); 60-80 bpm; 80-100 bpm; 100-120 bpm

vorzunehmen. Dies ist insbesondere dann vorteilhaft, wenn eine natürliche Variation eines Zustandsparameters beobachtet wird, dessen Schwankungsbereich nicht feststeht, z.B. die Herzfrequenz. Die erfassten Werte des physiologischen Parameters werden getrennt in den verschiedenen Bereichen des Zustandsparameters gemittelt. In regelmäßigen Intervallen (z.B. alle 24 h) kann dieser Datensatz ausgewertet werden. Erst dann wird das erste Differenzsignal in Form der Wertänderung des physiologischen Parameters zwischen verschiedenen Zustandsbereichen berechnet, also als Differenz von Bin-Mittelwerten, und kann auch mit den dazugehörigen Wertänderungen des Zustandsparameters (z.B. Zustandsparameter-Differenz zwischen den Zustandsbereichen, Bin-Abstand) in Beziehung gesetzt werden. Dies ermöglicht das Mitteln von erfassten Werten, ohne den Zustandsparameter kontrollieren zu können oder dessen Variationsbereich zu kennen.

Beispielsweise bestimmt die Auswerteeinheit die Wertänderung des physiologischen Parameters aus den gespeicherten Bin-Mittelwerten des physiologischen Parameters für Wertebereiche des Zustandsparameters nach einer der folgenden Methoden:

- Berechnen der Breite des von den Bin-Mittelwerten des physiologischen Parameters überstrichenen Wertebereichs;

- Berechnen eines Mittelwerts von Mittelwertdifferenzen zwischen benachbarten Bins, bei variierendem Bin-Abstand jeweils normiert auf den Bin-Abstand;

- Berechnen eines auf den Bin-Abstand normierten Mittelwerts aller paarweisen Differenzen zwischen allen Bins und Gewichten dieses Mittelwerts mit der Anzahl der Erfassungsvorgänge; oder

- Berechnen einer Steigung einer ermittelten Fit-Kurve der Bin-Mittelwerte des physiologischen Parameters, aufgetragen gegen den Zustandsparameter.

Ein Variieren der Herzfrequenz kann beispielsweise aufgrund einer natürlichen Sinus-Frequenz selbständig erfolgen oder durch Frequenzanpassung mittels eines Implantats absichtlich verursacht werden. Ebenso kann ein Variieren einer atrioventrikulären Verzögerungszeit als alternativer Zustandsparameter aufgrund der intrinsischen atrioventrikulären Verzögerungszeit selbständig entstehen oder durch eine programmierte atrioventrikuläre Dynamik oder durch eine automatische atrioventrikuläre Nachführung absichtlich hervorgerufen werden.

In einer anderen Ausführungsform ist die Transformationseinheit der Überwachungsvorrichtung ausgebildet, das erste Differenzsignal zusätzlich in Abhängigkeit einer äußeren Bedingungen, wie beispielsweise "Ruhezustand" oder "Bewegungszustand", in die Kenngröße zu transformieren und die Vorhersageeinheit ebenfalls ausgebildet, die Kenngröße in Abhängigkeit der äußeren Bedingungen, wie beispielsweise "Ruhezustand des Patienten" oder "Bewegungszustand des Patienten", zu bewerten. Beispielsweise zeichnet die Auswerteeinheit der Überwachungsvorrichtung nur dann Werte des physiologischen Parameters auf, wenn eine bestimmte äußere Bedingung, wie beispielsweise "Ruhezustand des Patienten", erfüllt ist.

Durch diese getrennte Betrachtung der erfassten Werte des Zustandsparameters und des physiologischen Parameters erzielt die Überwachungsvorrichtung eine abermals erhöhte Spezifität.

In einer weiteren bevorzugten Ausführungsform der Überwachungsvorrichtung ist die Auswerteeinheit ausgebildet, eine zwischen Beginn der Wertänderung des Zustandsparameters und Ende der Wertänderung des physiologischen Parameters liegende Zeitdauer zu ermitteln. In dieser Ausführungsform ist die Transformationseinheit ausgebildet, das erste Differenzsignal auf die ermittelte Zeitdauer zu beziehen und das zeitbezogene erste Differenzsignal als die Kenngröße bereitzustellen. Alternativ stellt die Transformationseinheit die ermittelte Zeitdauer selbst als Kenngröße bereit.

In dieser Ausführungsform beschreibt die Kenngröße demnach eine Dynamik einer Reaktion in Form der auf die Wertänderung des Zustandsparameters hin erfolgenden Wertänderung des physiologischen Parameters. Diese Ausführungsform ist insbesondere dann vorteilhaft, wenn die Wertänderung des Zustandsparameters plötzlich erfolgt, wie dies beispielsweise bei einem Auftreten einer Extrasystole oder einem Entstehen von Vorhofflimmern der Fall ist.

In einer besonders bevorzugten Ausführungsform umfasst die Überwachungsvorrichtung zusätzlich implantierbare erste Mittel zum Verursachen einer absichtlichen Wertänderung des Zustandsparameters. Die Überwachungsvorrichtung ist demnach ausgebildet, die Wertänderung des physiologischen Parameters durch absichtliche Wertänderung des Zustandsparameters zu provozieren. Der Zustandsparameter ist in dieser Ausführungsform bevorzugt ein einen Zustand einer mit dem Patienten bereits verbundenen medizinischen Vorrichtung, wie beispielsweise ein Herzschrittmacher, ein Kardioverter, ein Defibrillator, eine CRT-Vorrichtung, charakterisierender Parameter.

Demnach umfasst die Überwachungsvorrichtung beispielsweise bevorzugt implantierbare erste Mittel zum Variieren einer Stimulationsfrequenz, Mittel zum Variieren der atrioventrikulären oder interventrikulären Verzögerungszeit, Mittel zum Variieren des Stimulationsmodus oder Mittel zum Aktivieren und Deaktivieren einer kardialen Resynchronisationstherapie. In dieser Ausführungsform transformiert die Transformationseinheit das erste Differenzsignal bevorzugt in Abhängigkeit der zeitlichen Korrelation der Wertänderungen des Zustandsparameters und des physiologischen Parameters.

Vor dem Hintergrund der Erkenntnis, dass das Herz-Kreislauf-System des Patienten auf bestimmte Störungen anders reagiert, wenn sich eine kardiovaskuläre Unregelmäßigkeit, wie beispielsweise eine kardiale Dekompensation, anbahnt, wird das Herz-Kreislauf-System des Patienten bewusst von der Überwachungsvorrichtung durch Verursachen einer absichtlichen Wertänderung des Zustandsparameters gestört und die Reaktion in Form der Wertänderung des physiologischen Parameters erfasst und von der Transformationseinheit in Abhängigkeit der zeitlichen Korrelation der Wertänderungen des Zustandsparameters und des physiologischen Parameters in eine Kenngröße transformiert und von der Vorhersageeinheit bewertet. Auf diese Weise ist die Überwachungsvorrichtung fähig, das für das Anbahnen einer kardiovaskulären Unregelmäßigkeit indikative Bewertungssignal sehr lange im Voraus und mit hoher Spezifität bereitzustellen und auszugeben.

In einer besonders bevorzugten Ausführungsform verursacht die Überwachungsvorrichtung wiederholend, beispielsweise alle 60 Minuten, für eine kurze Zeit, beispielsweise wenige Herzzyklen andauernd oder einige Minuten lang, eine bestimmte, bevorzugt sprunghafte, Wertänderung des Zustandsparameters und erfasst einen Wert des physiologischen Parameters vor, während und nach der Wertänderung des Zustandsparameters.

Bevorzugt ist die Auswerteeinheit in dieser Ausführungsform der Überwachungsvorrichtung ausgebildet, das erste Differenzsignal als Mittelwert einer Vielzahl bestimmter Wertänderungen des physiologischen Parameters, die auf eine Vielzahl detektierter Wertänderungen des Zustandsparameters hin erfolgten, bereitzustellen. Auf diese Weise schlagen sich Wertänderungen des physiologischen Parameters, welche auf andere Gründe als die Wertänderung des Zustandsparameters zurückzuführen sind, kaum im ersten Differenzsignal nieder, womit letztlich die Spezifität der Überwachungsvorrichtung weiter erhöht ist.

Bevorzugt ist auch die Transformationseinheit ausgebildet, die Kenngröße über einen bestimmten Zeitraum zeitlich zu mitteln, beispielsweise über 24 Stunden, und der Vorhersageeinheit diese zeitlich gemittelte Kenngröße zum Bewerten bereitzustellen. Durch das zeitliche Mitteln wird der Einfluss auf die Wertänderung des physiologischen Parameters anderer Ursachen als der Wertänderung des Zustandsparameters reduziert und somit die Spezifität der Überwachungsvorrichtung weiter erhöht.

Der Betrag der absichtlichen Wertänderung des Zustandsparameters ist beispielsweise vordefiniert oder wird von der Überwachungsvorrichtung adaptiv in Abhängigkeit der bestimmten Wertänderung des physiologischen Parameters festgelegt.

Die Kenngröße ist beispielsweise eine auf die Wertänderung des Zustandsparameters normierte Differenz eines Wertes des physiologischen Parameters unmittelbar vor der Wertänderung des Zustandsparameters von einem Wert des physiologischen Parameters an einem festgelegten Ende der Wertänderung des Zustandsparameters.

In einer anderen Ausführungsform ist die Kenngröße beschreibend für eine Dynamik der Reaktion in Form der auf die Wertänderung des Zustandsparameters hin erfolgenden Wertänderung des physiologischen Parameters. In dieser Ausführungsform transformiert die Transformationseinheit das erste Differenzsignal bevorzugt in Abhängigkeit der zeitlichen Korrelation der Wertänderungen des Zustandsparameters und des physiologischen Parameters in die Kenngröße. Beispielsweise setzt die Transformationseinheit das erste Differenzsignal, beispielsweise in Form der Wertänderung des physiologischen Parameters selbst, in Relation zu einer Reaktionszeit, die beispielsweise definiert ist als Halbwertszeit oder als Relaxationszeit einer abklingenden Exponentialfunktion, oder in Relation zu einer initialen Wertänderungsgeschwindigkeit des physiologischen Parameters, also in Relation zu einer Anfangssteigerung des Wertes des physiologischen Parameters. Andererseits kann die Reaktionszeit, die Halbwertszeit, die Relaxationszeit oder die Anfangssteigung selbst die Kenngröße sein.

Andere Beispiele für die Kenngröße sind eine auf die Wertänderung des Zustandsparameters normierte initiale Wertänderungsgeschwindigkeit des physiologischen Parameters, die Höhe eines Überschwingers über einen endgültigen Gleichgewichtszustand oder die Höhe des Überschwingers über den endgültigen Gleichgewichtszustand normiert auf die Wertänderung des Zustandsparameters.

In einer anderen Ausführungsform der Überwachungsvorrichtung verursachen die implantierbaren ersten Mittel lediglich eine einmalige kurzzeitige Wertänderung des Zustandsparameters, beispielsweise ein kurz andauerndes Erhöhen der Stimulationsfrequenz oder ein Verursachen einer künstlichen "Extrasystole", z.B. durch eine vorzeitige Stimulation. In dieser Ausführungsform ist die Kenngröße beispielsweise die auf die Wertänderung des Zustandsparameters bezogene Stärke und Richtung der durch die Wertänderung des Zustandsparameters verursachten Wertänderung des physiologischen Parameters, beispielsweise ein Herzraten-Sprung bei einer absichtlich verursachten Extrasystole. Andere bevorzugte Beispiele für die Kenngröße in dieser Ausführungsform der Überwachungsvorrichtung ist die bestimmte Wertänderung des physiologischen Parameters bezogen auf eine als Halbwertszeit oder Relaxationszeit einer abklingenden Exponentialfunktion definierten Reaktionszeit oder bezogen auf eine initiale Wertänderungsgeschwindigkeit des physiologischen Parameters oder die auf eine Amplitude und Dauer der Wertänderung des Zustandsparameters normierte initiale Wertänderungsgeschwindigkeit, die Höhe eines ersten Überschwingers, die auf die Amplitude und Dauer der Wertänderung des Zustandsparameters normierte Höhe des ersten Überschwingers, die Höhe eines zweiten Überschwingers oder die auf die Amplitude und Dauer der Wertänderung des Zustandsparameters normierte Höhe des zweiten Überschwingers. Andererseits kann auch die Amplitude und Dauer der Wertänderung des physiologischen Parameters, die Reaktionszeit, die Halbwertszeit, die Relaxationszeit, die Anfangssteigung, die Höhe des ersten Überschwingers oder die Höhe des zweiten Überschwingers selbst die Kenngröße sein.

Mittels absichtlich herbeigeführter Wertänderungen des Zustandsparameters, die nur wenige Herzzyklen andauern, überprüft die Überwachungsvorrichtung vergleichsweise schnelle Kompensationsmechanismen des Patienten, wie beispielsweise den Frank-Starling-Effekt, Bowditch-Effekt, eine autonome Regulation, während die Überwachungsvorrichtung mittels solcher absichtlich herbeigeführter Wertänderungen des Zustandsparameters, die einige Minuten bis einige Stunden andauern, regulatorische Kompensationsfähigkeiten des gesamten Herz-Kreislauf-Systems des Patienten überprüft.

In einer bevorzugten Ausführungsform der Überwachungsvorrichtung ist der implantierbare Sensor ausgebildet, einen Wert eines hämodynamischen Parameters als physiologischen Parameter zu erfassen, bevorzugt eines solchen hämodynamischen Parameters, welcher sich früh in der Wirkungskette einer kardialen Dekompensation ändert. Die Detektionseinheit der Überwachungsvorrichtung ist bevorzugt ausgebildet, eine Änderung eines Stimulationsmodus einer mit dem Patienten bereits verbundenen medizinischen Vorrichtung als Wertänderung eines Zustandsparameters zu detektieren.

In einer besonders bevorzugten Ausführung der Überwachungsvorrichtung erfasst die Detektionseinheit die natürliche (intrinsische oder frequenzadaptierte) Herzfrequenz als Zustandsparameter. Als physiologischer Parameter dient ein aus einer geeigneten Impedanzmessung abgeleiteter Surrogatparameter für das Schlagvolumen oder das endsystolische Volumen. Die Auswerteeinheit mittelt Werte des physiologischen Parameters in einem Vektor von Bins für vorbestimmte Herzfrequenzintervalle, bspw. 40-60, 60-80, 80-100, 100-120, 120-140, 140-180 bpm, und wertet den physiologischen Parameter alle 24 h aus. Die Kenngröße ist die mittlere Differenz aller benachbarten Herzfrequenzbins. Die Transformationseinheit berechnet die Kenngröße durch eine statistische Analyse der Trendwahrscheinlichkeit. Die Vorhersageeinheit vergleicht die Kenngröße mit einem Grenzwert. Wahlweise kann anstelle des Impedanz-Surrogatparameters für das Schlagvolumen oder das endsystolische Volumen der mit einem in der Pulmonalarterie implantierten Drucksensor gemessene pulmonalarterielle Druck verwendet werden (als Surrogatparameter für den linksventrikulären enddiastolischen Druck); oder ein Schlagvolumen durch Pulskonturanalyse aus dem pulmonalarteriellen Druck gewonnen werden.

In einer anderen besonders bevorzugten Ausführung verursachen die implantierbaren ersten Mittel der Überwachungsvorrichtung wiederholend eine künstliche Erhöhung der Stimulationsfrequenz als Zustandsparameter um einen bestimmten Wert, beispielsweise um 10 bpm über den Normalwert für 60 Herzzyklen einmal pro Stunde. Diese Provokation der Herzfrequenzerhöhung erfolgt nur bei niedrigen Herzfrequenzen, wie beispielsweise 40 - 90 bpm und bei Ruhe (kein Motion-Signal) des Patienten. Als physiologischer Parameter dient ein aus einer geeigneten Impedanzmessung abgeleiteter Surrogatparameter für das Schlagvolumen oder das endsystolische Volumen. Die Auswerteeinheit bestimmt das erste Differenzsignal, beispielsweise die Wertänderung des Schlagvolumens oder die Wertänderung des endsystolischen Volumens, sofort nach jeder Provokation und mittelt diese über einen längeren Zeitraum, wie 24 h. Die Kenngröße ist die mittlere Wertänderung des physiologischen Parameters. Die Vorhersageeinheit bewertet die Kenngröße durch Vergleich mit der über die lange Zeit gemittelten statistischen Schwankungsbreite bewertet. Wahlweise kann anstelle des Impedanz-Surrogatparameters für das Schlagvolumen oder das endsystolische Volumen der mit einem in der Pulmonalarterie implantierten Drucksensor gemessene pulmonalarterielle Druck verwendet werden (als Surrogatparameter für den linksventrikulären enddiastolischen Druck); oder ein Schlagvolumen durch Pulskonturanalyse aus dem pulmonalarteriellen Druck gewonnen werden.

In einer weiteren bevorzugten Ausführungsform der Überwachungsvorrichtung ist die Transformationseinheit ausgebildet, das erste Differenzsignal in Abhängigkeit der zeitlichen Korrelation der Wertänderungen des Zustandsparameters und des physiologischen Parameters und/oder in Abhängigkeit des Betrages und/oder der Richtung der detektierten Wertänderung des Zustandsparameters in eine Vielzahl Kenngrößen zu transformieren und diese bereitzustellen. Ferner ist in dieser Ausführungsform die Vorhersageeinheit ausgebildet, die Vielzahl Kenngrößen zu empfangen und zu bewerten und in Abhängigkeit der Vielzahl Bewertungen das für ein Anbahnen einer kardiovaskulären Unregelmäßigkeit indikative Bewertungsergebnissignal bereitzustellen.

Ein Teil der Vielzahl Kenngrößen ist in dieser Ausführungsform bevorzugt beschreibend für eine Empfindlichkeit einer Reaktion in Form der auf eine Wertänderung des Zustandsparameters hin erfolgenden Wertänderung des physiologischen Parameters und ein anderer Teil der Vielzahl Kenngrößen beschreibend für eine Dynamik dieser Reaktion. Durch das Bewerten der Vielzahl Kenngrößen mittels der Vorhersageeinheit weist die Überwachungsvorrichtung eine abermals verbesserte Spezifität und längere Vorwarnzeit auf.

In einer weiteren bevorzugten Ausführungsform ist die Vorhersageeinheit weiter ausgebildet, aus der Kenngröße eine Soll-Wertänderung des Zustandsparameters zu bestimmen, wobei eine tatsächliche Wertänderung des Zustandsparameters gemäß der bestimmten Soll-Wertänderung einer sich anbahnenden kardiovaskulären Unregelmäßigkeit entgegenwirkt.

Bevorzugt sind die implantierbaren ersten Mittel der Überwachungsvorrichtung dann ausgebildet, eine tatsächliche Wertänderung des Zustandsparameters gemäß der bestimmten Soll-Wertänderung zu verursachen. In dieser Ausführungsform ist der Zustandsparameter beispielsweise eine atrioventrikuläre Verzögerungszeit. Zunächst verursacht die Überwachungsvorrichtung eine Wertänderung der atrioventrikulären Verzögerungszeit, um so eine Wertänderung eines hämodynamischen Parameters zu erfassen und letztlich die Kenngröße zu generieren. Die Vorhersageeinheit bestimmt aus der Kenngröße die Soll-Wertänderung des Zustandsparameters, welche einer kardiovaskulären Unregelmäßigkeit entgegenwirken würde, beispielsweise eine bestimmte Verlängerung der atrioventrikulären Verzögerungszeit. Die implantierbaren ersten Mittel verursachen dann beispielsweise ein entsprechendes Variieren der atrioventrikulären Verzögerungszeit.

Einen zweiten Aspekt der vorliegenden Erfindung bildet eine implantierbare Elektrotherapievorrichtung, die mit einer erfindungsgemäßen Überwachungsvorrichtung wirkverbunden ist und zweite Mittel zum Ausgeben eines elektrischen Therapiesignals umfasst. Die implantierbare Elektrotherapievorrichtung teilt die Vorteile der erfindungsgemäßen Überwachungsvorrichtung des ersten Aspektes der Erfindung.

Bevorzugt ist die implantierbare Elektrotherapievorrichtung ausgebildet, das elektrische Therapiesignal in Abhängigkeit des Bewertungsergebnissignals der Überwachungsvorrichtung auszugeben.

Die implantierbare Elektrotherapievorrichtung ist beispielsweise ein Herzschrittmacher, ein Kardioverter, ein Defibrillator oder ein kombinierter Kardioverter/Defibrillator.

In einer besonders bevorzugten Ausführungsform ist die implantierbare Elektrotherapievorrichtung ein mit einer erfindungsgemäßen Überwachungsvorrichtung verbundenes CRT-Implantat mit automatischer AVD/VVD-Anpassung. Die automatische AVD/VVD-Anpassung variiert in regelmäßigen Abständen die AV-Zeit und/oder die VV-Zeit, so dass nach einer bestimmten Zeit alle erlaubten Kombinationen von AVD und VVD-Werten erfasst wurden. Dieser Mechanismus wird zusätzlich zur Überwachung des Patienten eingesetzt. Als Zustandsparameter dient die Kombination aus AVD und VVD. Als physiologischer Parameter dient ein aus einer geeigneten Impedanzmessung abgeleiteter Surrogatparameter für das Schlagvolumen oder das endsystolische Volumen. Die Auswerteeinheit der Überwachungsvorrichtung des CRT-Implantats mittelt Werte des physiologischen Parameters in einer Matrix von Bins für alle möglichen AVD/VVD-Kombinationen und wertet diese regelmäßig, beispielsweise alle 24 h, aus. Die Kenngröße ist die Breite des überstrichenen Bereichs des in der Matrix gespeicherten Wertes des physiologischen Parameters. Die Kenngröße wird von der Auswerteeinheit durch eine statistische Analyse der Trendwahrscheinlichkeit berechnet und von der Vorhersageeinheit mit einem Grenzwert verglichen.

Einen dritten Aspekt der Erfindung bildet ein Verfahren zum Betreiben einer Überwachungsvorrichtung, das die folgenden Schritte umfasst:

- Erfassen eines Wertes einer von einer kardiovaskulären Unregelmäßigkeit abhängigen physiologischen Parameters;

- Detektieren einer Wertänderung eines vom physiologischen Parameter verschiedenen Zustandsparameters, wobei der Zustandsparameter ein anderer physiologischer Parameter oder ein einen Zustand einer mit dem Patienten bereits verbundenen medizinischen Vorrichtung charakterisierender Parameter oder ein einen sonstigen physischen oder pharmakotherapeutischen Zustand des Patienten charakterisierender Parameter ist;

- auf die Detektion der Wertänderung des Zustandsparameters hin: Bestimmen einer auf die Wertänderung des Zustandsparameters hin erfolgenden Wertänderung des physiologischen Parameters und Bereitstellen eines ersten Differenzsignals in Abhängigkeit der erfassten Wertänderung des physiologischen Parameters;

- Transformieren des Differenzsignals in Abhängigkeit einer zeitlichen Korrelation der Wertänderungen des Zustandsparameters und des physiologischen Parameters und/oder in Abhängigkeit eines Betrages und/oder einer Richtung der detektierten Wertänderung des Zustandsparameters in eine Kenngröße und Bereitstellen der Kenngröße;

- Bewerten der Kenngröße und Bereitstellen eines für ein Anbahnen einer kardiovaskulären Unregelmäßigkeit indikativen Bewertungsergebnissignals in Abhängigkeit der Bewertung.

Das Verfahren zum Betreiben einer Überwachungsvorrichtung des dritten Aspektes der Erfindung teilt die Vorteile der Überwachungsvorrichtung des ersten Aspektes der Erfindung.

Nachfolgend werden weitere Vorteile der vorliegenden Erfindung durch Beschreiben einiger Ausführungsbeispiele der Erfindung anhand der Zeichnungen erläutert. In den Zeichnungen zeigen in schematischer Darstellung

Figur 1:  eine bevorzugte Ausführungsform der Überwachungsvorrichtung des ersten Aspektes der Erfindung;

Figur 2:  eine bevorzugte Ausführungsform der implantierbaren Elektrotherapievorrichtung des zweiten Aspektes der Erfindung;

Figur 3:  ein Ablaufdiagramm einer bevorzugten Ausführungsform des Verfahrens zum Betreiben einer Überwachungsvorrichtung gemäß dem dritten Aspekt der Erfindung.

Vorausschickend sei bemerkt, dass die in den Figuren gezeigte geometrische Anordnung der Komponenten der Überwachungsvorrichtung oder der Elektrotherapievorrichtung in keinem bestimmten Zusammenhang zu einem tatsächlichen Aufbau der Überwachungsvorrichtung bzw. der Elektrotherapievorrichtung steht.

Die Komponenten der Überwachungsvorrichtung müssen insbesondere nicht allesamt in einem Gehäuse integriert sein, sondern können räumlich verteilt angeordnet sein. Bevorzugt sind beispielsweise der erste Eingang und der Sensor der Überwachungsvorrichtung implantiert, die Detektionseinheit mit dem zweiten Eingang in Abhängigkeit des Typs des Zustandsparameters wahlweise ebenfalls implantiert oder außerhalb des Körpers des Patienten angeordnet und die übrigen Komponenten, nämlich die Auswerteeinheit, die Transformationseinheit und die Vorhersageeinheit außerhalb des Körpers des Patienten, beispielsweise in einem Patientengerät oder in einem Home-Monitoring-Center, befindlich. Der implantierte Sensor und die Detektionseinheit senden dann ihre jeweiligen Signale über geeignete Schnittstellen zur Auswerteeinheit.

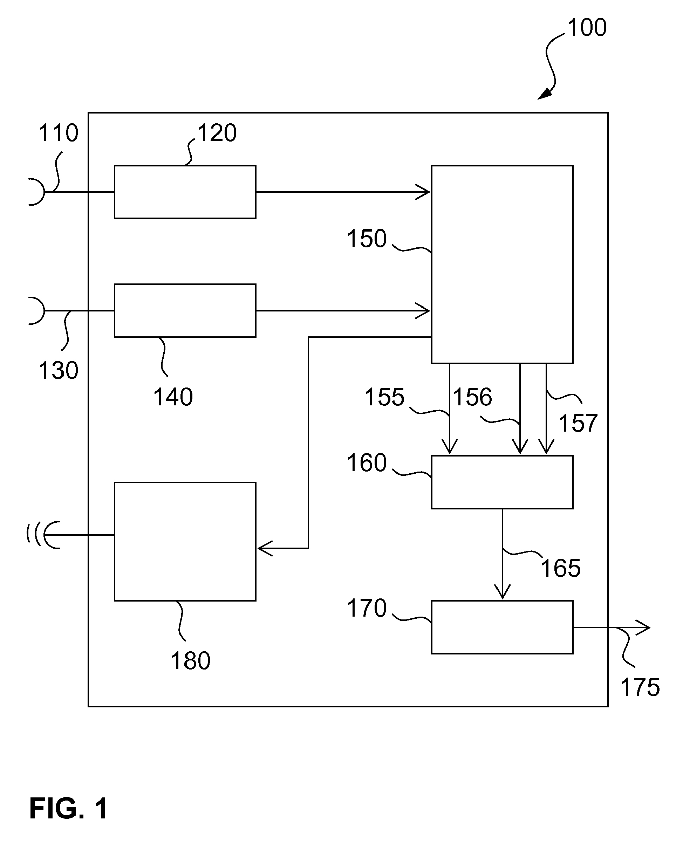

Fig. 1 zeigt in schematischer Darstellung eine bevorzugte Ausführungsform (100) der erfindungsgemäßen Überwachungsvorrichtung für einen Patienten zum Vorhersagen einer kardiovaskulären Unregelmäßigkeit. Die Überwachungsvorrichtung 100 umfasst einen ersten Eingang 110 und einen mit dem ersten Eingang 110 verbundenen Sensor 120 zum Erfassen eines Wertes eines von einer kardiovaskulären Unregelmäßigkeit abhängigen physiologischen Parameters. Der erste Eingang 110 und der Sensor 120 sind implantierbar. Die Überwachungsvorrichtung 100 umfasst weiter einen zweiten Eingang 130 und eine mit dem zweiten Eingang 130 verbundene Detektionseinheit 140 zum Detektieren einer Wertänderung eines vom physiologischen Parameter verschiedenen Zustandsparameters. Die Detektionseinheit 140 und der zweite Eingang 130 sind wahlweise implantiert oder befinden sich außerhalb des Patienten.

Insofern stellt beispielsweise der in Fig. 1 gezeigte Rahmen um die Vielzahl Komponenten der Überwachungsvorrichtung 100 kein Implantatsgehäuse dar, sondern dient primär der Verdeutlichung, dass alle in Fig. 1 gezeigten Komponenten Teil dieser Ausführungsform der Überwachungsvorrichtung 100 sind.

In Abhängigkeit der bestimmten Wertänderung des physiologischen Parameters und der detektierten Wertänderung des Zustandsparameters stellt die Überwachungsvorrichtung 100 ein für ein Anbahnen einer kardiovaskulären Unregelmäßigkeit indikatives Bewertungsergebnissignal 175 bereit.

Mit dem implantierbaren Sensor 120 und der Detektionseinheit 140 ist eine Auswerteeinheit 150 wirkverbunden, welche ausgebildet ist, auf die Detektion der Wertänderung des Zustandsparameters hin eine auf die Wertänderung des Zustandsparameters hin erfolgende Wertänderung des physiologischen Parameters zu bestimmen und in Abhängigkeit von der bestimmten Wertänderung des physiologischen Parameters ein erstes Differenzsignal 155 bereitzustellen. Das erste Differenzsignal 155 kennzeichnet demnach die Wertänderung des physiologischen Parameters, die in der Regel in Reaktion auf eine Wertänderung des Zustandsparameters erfolgt. Wie bereits oben erläutert, kann sich die Auswerteeinheit 150 außerhalb des Patienten befinden, beispielsweise in einem Patientengerät oder in einem Home-Monitoring-Center. In diesem Fall empfängt die Auswerteeinheit 150 jeweilige Signale bevorzugt drahtlos.

Eine Transformationseinheit 160 empfängt das erste Differenzsignal 155 und transformiert das erste Differenzsignal 155 in wahlweiser Abhängigkeit einer zeitlichen Korrelation der Wertänderungen des Zustandsparameters und des physiologischen Parameters und/oder in Abhängigkeit eines Betrages und/oder einer Richtung der detektierten Wertänderung des Zustandsparameters in eine Kenngröße 165. Durch die Transformation des ersten Differenzsignals 155 in die Kenngröße 165 wird demnach mittels der Transformationseinheit die Wertänderung des physiologischen Parameters in Bezug gesetzt zu einem zeitlichen Verlauf der Wertänderungen des Zustandsparameters und des physiologischen Parameters, womit die Kenngröße 165 eine Dynamik der Reaktion beschreiben würde, oder in Bezug gesetzt auf die Wertänderung des Zustandsparameters selbst, womit die Kenngröße 165 kennzeichnend wäre für eine Empfindlichkeit der Reaktion.

Die Überwachungsvorrichtung 100 umfasst ferner eine Vorhersageeinheit 170, die ausgebildet ist, diese Kenngröße 165 zu empfangen und zu bewerten und in Abhängigkeit der Bewertung ein für ein Anbahnen einer kardiovaskulären Unregelmäßigkeit indikatives Bewertungsergebnissignal 175 bereitzustellen und auszugeben. Das Bewerten erfolgt beispielsweise durch Vergleichen mit bereits gespeicherten Vergleichsgrößen.

Wie oben erläutert, können sich die Transformationseinheit 160 und die Vorhersageeinheit außerhalb des Patienten befinden, beispielsweise, in einem Patientengerät oder in einem Home-Monitoring-Center.

Das Bewertungsergebnissignal 175 wird beispielsweise von einer weiteren (in Fig. 1 nicht näher dargestellten) Elektrotherapievorrichtung empfangen, welche ausgebildet ist, eine Therapie des Patienten in Abhängigkeit dieses Bewertungsergebnissignals 175 vorzunehmen.

Bei der in Fig. 1 dargestellten Ausführungsform der Überwachungsvorrichtung 100 ist die Auswerteeinheit 150 zusätzlich ausgebildet, die Wertänderung des Zustandsparameters zu bestimmen und in Abhängigkeit der bestimmten Wertänderung des Zustandsparameters ein zweites Differenzsignal 156 bereitzustellen. In dieser Ausführungsform ist der Zustandsparameter beispielsweise eine Herzfrequenz des Patienten oder eine atrioventrikuläre Verzögerungszeit. Die Transformationseinheit 160 empfängt auch das zweite Differenzsignal 156 und stellt in einer Ausführungsform die Kenngröße 165 als Quotienten aus dem ersten Differenzsignal 155 und dem zweiten Differenzsignal 156 bereit.

Die Auswerteeinheit 150 ist alternativ oder zusätzlich dazu ausgebildet, eine zwischen Beginn der Wertänderung des Zustandsparameters und Ende der Wertänderung des physiologischen Parameters liegende Zeitdauer zu ermitteln und der Transformationseinheit 160 als ermittelte Zeitdauer 157 bereitzustellen. Die Transformationseinheit 160 ist ausgebildet, das erste Differenzsignal 155 wahlweise auf das zweite Differenzsignal 156 zu beziehen und/oder auf die ermittelte Zeitdauer 157 und als Kenngröße 165 auszugeben. Die Kenngröße kann aber auch mit der ermittelten Zeitdauer 157 identisch sein.

Die Überwachungsvorrichtung 100 umfasst ferner mit der Auswerteeinheit 150 wirkverbundene implantierbare erste Mittel 180 zum Verursachen einer absichtlichen Wertänderung des Zustandsparameters. Wie bereits an anderer Stelle der Beschreibung erläutert, beruht die vorliegende Erfindung auf der Erkenntnis, dass das Herz-Kreislauf-System des Patienten auf eine Störung anders reagiert, wenn sich eine kardiovaskuläre Unregelmäßigkeit, wie beispielsweise eine kardiale Dekompensation, anbahnt. In diesem Sinne provoziert die Überwachungsvorrichtung 100 gezielte Störungen des Herz-Kreislauf-Systems des Patienten in Form von absichtlich herbeigeführten Wertänderungen eines Zustandsparameters mittels der implantierbaren ersten Mittel 180. Beispielsweise ist die Überwachungsvorrichtung 100 also ausgebildet, über die implantierbaren ersten Mittel 180 die Stimulationsfrequenz eines Herzschrittmachers des Patienten zu verändern oder eine kardiale Resynchronisationstherapie zu aktivieren bzw. zu deaktivieren oder die atrioventrikuläre Verzögerungszeit zu manipulieren. Eine solche Wertänderung des Zustandsparameters detektiert die Detektionseinheit 140. Die auf die Wertänderung des Zustandsparameters hin erfolgende Wertänderung des physiologischen Parameters, beispielsweise ein hämodynamischer Parameter, wird von dem implantierbaren Sensor 120 und von der Auswerteeinheit 150 erfasst und bestimmt.

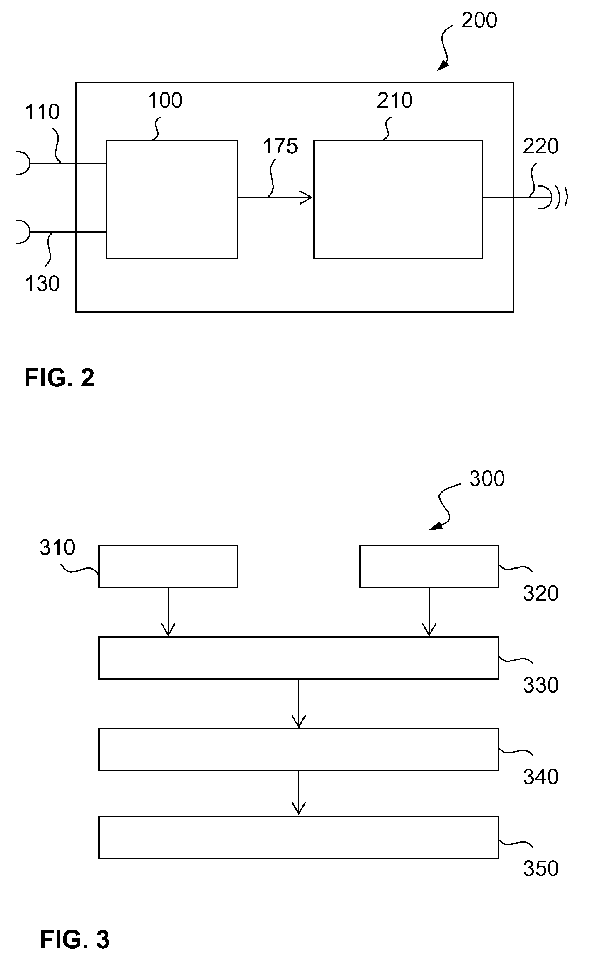

Fig. 2 zeigt eine bevorzugte Ausführungsform der erfindungsgemäßen implantierbaren Elektrotherapievorrichtung. Die implantierbare Elektrotherapievorrichtung 200 ist mit einer erfindungsgemäßen Überwachungsvorrichtung 100, umfassend einen ersten Eingang 110 zum Erfassen eines Wertes eines physiologischen Parameters und einen zweiten Eingang 130 zum Detektieren einer Wertänderung eines Zustandsparameters wirkverbunden. Wie zu Fig. 1 ausgeführt, erzeugt die Überwachungsvorrichtung 100 mit Hilfe einer Auswerteeinheit, einer Transformationseinheit und einer Vorhersageeinheit ein Bewertungsergebnissignal 175 in Abhängigkeit der erfassten Wertänderung des physiologischen Parameters und beispielsweise der erfassten Wertänderung des Zustandsparameters. Die implantierbare Elektrotherapievorrichtung 200 umfasst ferner zweite Mittel 210 zum Ausgeben eines elektrischen Therapiesignals 220. Das Therapiesignal 220 ist beispielsweise ein Stimulationsimpuls. Bevorzugt sind die zweiten Mittel 210 ausgebildet, das elektrische Therapiesignal in Abhängigkeit des Bewertungsergebnissignals 175 auszugeben.

Die implantierbare Elektrotherapievorrichtung 200 ist beispielsweise ein Herzschrittmacher, ein implantierbarer Kardioverter oder ein implantierbarer Defibrillator.

Fig. 3 zeigt in schematischer Darstellung ein Ablaufdiagramm einer bevorzugten Ausführungsform des erfindungsgemäßen Verfahrens zum Betreiben einer Überwachungsvorrichtung gemäß dem dritten Aspekt der vorliegenden Erfindung. Gemäß diesem Verfahren 300 wird in einem ersten Schritt 310 fortwährend ein Wert eines von einer kardiovaskulären Unregelmäßigkeit abhängigen physiologischen Parameters erfasst.

In einem zweiten Schritt 320 wird eine Wertänderung eines vom physiologischen Parameter verschiedenen Zustandsparameters detektiert.

Auf die Detektion der Wertänderung des Zustandsparameters hin erfolgt in einem dritten Schritt 330 ein Bestimmen einer auf die Wertänderung des Zustandsparameters hin erfolgende Wertänderung des physiologischen Parameters und ein Bereitstellen eines ersten Differenzsignals in Abhängigkeit der erfassten Wertänderung des physiologischen Parameters.

In einem vierten Schritt 340 erfolgt ein Transformieren des Differenzsignals in Abhängigkeit einer zeitlichen Korrelation der Wertänderung des Zustandsparameters und des physiologischen Parameters und/oder in Abhängigkeit eines Betrages und/oder einer Richtung der detektierten Wertänderung des Zustandsparameters in eine Kenngröße und ein Bereitstellen der Kenngröße.

Schließlich erfolgt in einem fünften Schritt 350 ein Bewerten dieser Kenngröße und ein Bereitstellen eines für ein Anbahnen einer kardiovaskulären Unregelmäßigkeit indikativen Bewertungsergebnissignals in Abhängigkeit der Bewertung.

Die vorliegende Erfindung betrifft eine Überwachungsvorrichtung für einen Patienten zum Vorhersagen einer kardiovaskulären Unregelmäßigkeit sowie ein Verfahren zum Betreiben einer Überwachungsvorrichtung. Ferner betrifft die vorliegende Erfindung eine implantierbare Elektrotherapievorrichtung, wie beispielsweise ein implantierbarer Herzschrittmacher, ein implantierbarer Kardioverter oder ein implantierbarer Defibrillator, mit einer erfindungsgemäßen Überwachungsvorrichtung. Die erfindungsgemäße Überwachungsvorrichtung erfasst in einer Ausführungsform eine Wertänderung eines hämodynamischer Parameters, die in Folge einer detektierten Wertänderung eines Zustandsparameters, wie beispielsweise in Folge eines Aktivierens oder Deaktivierens einer kardialen Resynchronisationstherapie, stattfindet. Durch geeignete Bewertung der Wertänderung des hämodynamischen Parameters kann die Überwachungsvorrichtung ein für ein Anbahnen einer kardiovaskulären Unregelmäßigkeit, wie beispielsweise eine kardiale Dekompensation, indikatives Bewertungsergebnissignal lange im Voraus und mit hoher Spezifität ausgeben.

**Patentansprüche**

1. Überwachungsvorrichtung (100) für einen Patienten zum Vorhersagen einer kardiovaskulären Unregelmäßigkeit, umfassend:

    - einen ersten Eingang (110) und einen mit dem ersten Eingang (110) verbundenen Sensor (120) zum Erfassen eines Wertes eines von einer kardiovaskulären Unregelmäßigkeit abhängigen physiologischen Parameters, wobei der erste Eingang (110) und der Sensor (120) implantierbar sind,
    - einen zweiten Eingang (130) und eine mit dem zweiten Eingang (130) verbundene Detektionseinheit (140) zum Detektieren einer Wertänderung eines vom physiologischen Parameter verschiedenen Zustandsparameters, wobei der Zustandsparameter ein anderer physiologischer Parameter oder ein einen Zustand einer mit dem Patienten bereits verbundenen medizinischen Vorrichtung charakterisierender Parameter oder ein einen sonstigen physischen oder pharmakotherapeutischen Zustand des Patienten charakterisierender Parameter ist,
    - eine mit dem implantierbaren Sensor (120) und der Detektionseinheit (140) wirkverbundene Auswerteeinheit (150), die ausgebildet ist, auf die Detektion der Wertänderung des Zustandsparameters hin eine auf die Wertänderung des Zustandsparameters hin erfolgende Wertänderung des physiologischen Parameters zu bestimmen und in Abhängigkeit der bestimmten Wertänderung des physiologischen Parameters ein erstes Differenzsignal (155) bereitzustellen,
    - eine Transformationseinheit (160), die ausgebildet ist, das erste Differenzsignal (155) zu empfangen und in Abhängigkeit einer zeitlichen Korrelation der Wertänderungen des Zustandsparameters und des physiologischen Parameters und/oder in Abhängigkeit eines Betrages und/oder einer Richtung der detektierten Wertänderung des Zustandsparameters in eine Kenngröße (165) zu transformieren und bereitzustellen,
    - eine Vohersageeinheit (170), die ausgebildet ist, die Kenngröße (165) zu empfangen und zu bewerten sowie in Abhängigkeit der Bewertung ein für ein Anbahnen einer kardiovaskulären Unregelmäßigkeit indikatives Bewertungsergebnissignal (175) bereitzustellen.

2. Überwachungsvorrichtung (100) nach Anspruch 1, bei der

    - die Auswerteeinheit (150) ausgebildet ist, die Wertänderung des Zustandsparameters zu bestimmen und in Abhängigkeit der bestimmten Wertänderung des Zustandsparameters ein zweites Differenzsignal (156) bereitzustellen und
    - die Transformationseinheit (160) ausgebildet ist, einen Quotienten aus dem ersten Differenzsignal (155) und dem zweiten Differenzsignal (156) als die Kenngröße (165) bereitzustellen.

3. Überwachungsvorrichtung (100) nach einem der vorstehenden Ansprüche, bei der die Auswerteeinheit (150) ausgebildet ist,

    - kontinuierlich den Wert des physiologischen Parameters aufzuzeichnen und für kurze Zeit zeitlich zu mitteln sowie den kurzzeitgemittelten Wert des physiologischen Parameters zu speichern und kontinuierlich zu aktualisieren und
    - die Wertänderung des physiologischen Parameters als Differenz zwischen einem aktuell erfassten Wert des physiologischen Parameters und dem zuvor gespeicherten Wert des physiologischen Parameters zu bestimmen.

4. Überwachungsvorrichtung (100) nach einem der vorstehenden Ansprüche, bei der

    - die Auswerteeinheit (150) ausgebildet ist, eine zwischen Beginn der Wertänderung des Zustandsparameters und Ende der Wertänderung des physiologischen Parameters liegende Zeitdauer zu ermitteln und
    - die Transformationseinheit (160) ausgebildet ist, das erste Differenzsignal (155) auf die ermittelte Zeitdauer (157) zu beziehen und das zeitbezogene erste Differenzsignal als die Kenngröße (165) bereitzustellen.

5. Überwachungsvorrichtung (100) nach einem der vorstehenden Ansprüche, zusätzlich umfassend implantierbare erste Mittel (180) zum Verursachen einer absichtlichen Wertänderung des Zustandsparameters.

6. Überwachungsvorrichtung (100) nach Anspruch 5, bei der der implantierbare Sensor (120) und die Auswerteeinheit (150) zum Bestimmen der Wertänderung des physiologischen Parameters ausgebildet sind, einen Wert des physiologischen Parameters jeweils vor, während und nach der absichtlichen Wertänderung des Zustandsparameters zu erfassen.

7. Überwachungsvorrichtung (100) nach Anspruch 6, bei der die Auswerteeinheit (150) ausgebildet ist, das erste Differenzsignal (155) als Mittelwert einer Vielzahl bestimmter Wertänderungen des physiologischen Parameters, die auf eine Vielzahl detektierter Wertänderungen des Zustandsparameters hin erfolgten, bereitzustellen.

8. Überwachungsvorrichtung (100) nach einem der vorstehenden Ansprüche, bei der der implantierbare Sensor (120) ausgebildet ist, einen Wert eines hämodynamischen Parameters als physiologischen Parameter zu erfassen.

9. Überwachungsvorrichtung (100) nach einem der vorstehenden Ansprüche, bei der die Detektionseinheit (140) ausgebildet ist, eine Änderung eines Stimulationsmodus' einer mit dem Patienten bereits verbundenen medizinischen Vorrichtung als Wertänderung eines Zustandsparameters zu detektieren.

10. Überwachungsvorrichtung (100) nach einem der vorstehenden Ansprüche, bei der

- die Transformationseinheit (160) ausgebildet ist, das erste Differenzsignal (155) in Abhängigkeit der zeitlichen Korrelation der Wertänderungen des Zustandsparameters und des physiologischen Parameters und/oder in Abhängigkeit des Betrages und/oder der Richtung der detektierten Wertänderung des Zustandsparameters in eine Vielzahl Kenngrößen (165) zu transformieren und diese bereitzustellen;
- die Vorhersageeinheit (170) ausgebildet ist, die Vielzahl Kenngrößen (165) zu empfangen und zu bewerten und in Abhängigkeit der Vielzahl Bewertungen das für ein Anbahnen einer kardiovaskulären Unregelmäßigkeit indikative Bewertungsergebnissignal (175) bereitzustellen.

11. Überwachungsvorrichtung (100) nach einem der vorstehenden Ansprüche, bei der die Transformationseinheit (160) ausgebildet ist, die Kenngröße zu mitteln und die zeitlich gemittelte Kenngröße (165) der Vorhersageeinheit (170) zum Bewerten bereitzustellen.

12. Implantierbare Elektrotherapievorrichtung (200), die mit einer Überwachungsvorrichtung (100) nach einem der Ansprüche 1 bis 11 wirkverbunden ist und zweite Mittel (210) zum Ausgeben eines elektrischen Therapiesignals (220) umfasst.

13. Herzschrittmacher (200), der mit einer Überwachungsvorrichtung (100) nach einem der Ansprüche 1 bis 11 wirkverbunden ist.

14. Implantierbarer Kardioverter (200) oder implantierbarer Defibrillator (200), der mit einer Überwachungsvorrichtung (100) nach einem der Ansprüche 1 bis 11 wirkverbunden ist.

15. Verfahren (300) zum Betreiben einer Überwachungsvorrichtung, umfassend die Schritte:

- Erfassen (310) eines Wertes eines von einer kardiovaskulären Unregelmäßigkeit abhängigen physiologischen Parameters;
- Detektieren (320) einer Wertänderung eines vom physiologischen Parameter verschiedenen Zustandsparameters, wobei der Zustandsparameter ein anderer physiologischer Parameter oder ein einen Zustand einer mit dem Patienten bereits verbundenen medizinischen Vorrichtung charakterisierender Parameter oder ein einen sonstigen physischen oder pharmakotherapeutischen Zustand des Patienten charakterisierender Parameter ist;
- auf die Detektion der Wertänderung des Zustandsparameters hin: Bestimmen (330) einer auf die Wertänderung des Zustandsparameters hin erfolgenden Wertänderung des physiologischen Parameters und Bereitstellen (330) eines ersten Differenzsignals in Abhängigkeit der erfassten Wertänderung des physiologischen Parameters;
- Transformieren (340) des Differenzsignals in Abhängigkeit einer zeitlichen Korrelation der Wertänderungen des Zustandsparameters und des physiologischen Parameters und/oder in Abhängigkeit eines Betrages und/ oder einer Richtung der detektierten Wertänderung des Zustandsparameters in eine Kenngröße und Bereitstellen (340) der Kenngröße;
- Bewerten (350) der Kenngröße und Bereitstellen (350) eines für ein Anbahnen einer kardiovaskulären Unregelmäßigkeit indikativen Bewertungsergebnissignals in Abhängigkeit der Bewertung.

**FIG. 1**

200

110

100

210

175

220

130

**FIG. 2**

300

310

320

330

340

350

**FIG. 3**

Europäisches
Patentamt

European
Patent Office

Office européen
des brevets

## EUROPÄISCHER RECHERCHENBERICHT

**Nummer der Anmeldung**

EP 11 15 5185

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,P | EP 2 241 249 A1 (BIOTRONIK CRM PATENT AG [CH]) 20. Oktober 2010 (2010-10-20) * das ganze Dokument * ----- | 1-15 | INV. A61B5/0205 A61N1/36 A61N1/38 |
| X | US 2009/163969 A1 (DONOFRIO WILLIAM T [US]) 25. Juni 2009 (2009-06-25) * Absätze [0048] - [0054] * * Abbildung 7 * * das ganze Dokument * ----- | 1-15 | |
| X,P | US 2010/094102 A1 (ZHANG YI [US] ET AL) 15. April 2010 (2010-04-15) * das ganze Dokument * ----- | 1-15 | |
| X | US 2010/023081 A1 (AUDET SARAH ANNE [US] ET AL) 28. Januar 2010 (2010-01-28) * das ganze Dokument * ----- | 1-15 | |
| A,D | US 2005/124900 A1 (STADLER ROBERT W [US] ET AL) 9. Juni 2005 (2005-06-09) * das ganze Dokument * ----- | 1-15 | RECHERCHIERTE SACHGEBIETE (IPC) A61B A61N |

Der vorliegende Recherchenbericht wurde für alle Patentansprüche erstellt

| Recherchenort | Abschlußdatum der Recherche | Prüfer |
|---|---|---|
| München | 25. Juli 2011 | Olapinski, Michael |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

.......................................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 11 15 5185

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-07-2011

| Im Recherchenbericht angeführtes Patentdokument | | Datum der Veröffentlichung | Mitglied(er) der Patentfamilie | | Datum der Veröffentlichung |
|---|---|---|---|---|---|
| EP 2241249 | A1 | 20-10-2010 | DE 102009002399 | A1 | 21-10-2010 |
| | | | US 2010268041 | A1 | 21-10-2010 |
| US 2009163969 | A1 | 25-06-2009 | WO 2009085818 | A2 | 09-07-2009 |
| US 2010094102 | A1 | 15-04-2010 | AU 2009302272 | A1 | 15-04-2010 |
| | | | WO 2010042790 | A2 | 15-04-2010 |
| US 2010023081 | A1 | 28-01-2010 | KEINE | | |
| US 2005124900 | A1 | 09-06-2005 | EP 1694201 | A1 | 30-08-2006 |
| | | | WO 2005055823 | A1 | 23-06-2005 |

EPO FORM P0461

Für nähere Einzelheiten zu diesem Anhang : siehe Amtsblatt des Europäischen Patentamts, Nr.12/82

**EP 2 364 640 A1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20050124900 A1